(19) Europäisches Patentamt
European Patent Office
Office européen des brevets

(11) EP 1 360 005 B1

(12) # EUROPÄISCHE PATENTSCHRIFT

(45) Veröffentlichungstag und Bekanntmachung des Hinweises auf die Patenterteilung:
**06.10.2004 Patentblatt 2004/41**

(21) Anmeldenummer: **02704673.9**

(22) Anmeldetag: **26.01.2002**

(51) Int Cl.[7]: **B01J 31/24**, C07F 9/6568, C07C 67/38, C07F 9/50

(86) Internationale Anmeldenummer:
**PCT/EP2002/000822**

(87) Internationale Veröffentlichungsnummer:
**WO 2002/064249 (22.08.2002 Gazette 2002/34)**

(54) **VON EINEM BIS-PHOSPHORINAN ABGELEITETE UND ZUR HERSTELLUNG EINES CARBONYLIERUNGS-KATALYSATORSYSTEMS GEEIGNETE VERBINDUNG**

COMPOUND SUITABLE FOR PRODUCING A CARBONYLATION CATALYST SYSTEM DERIVED FROM A BIS-PHOSPHORINANE

COMPOSE SERVANT A LA FABRICATION D'UN SYSTEME CATALYSEUR POUR LA CARBONYLATION DERIVE D'UN BIS-PHOSPHORINANE

(84) Benannte Vertragsstaaten:
**AT BE CH CY DE DK ES FI FR GB GR IE IT LI LU MC NL PT SE TR**

(30) Priorität: **09.02.2001 DE 10106348**

(43) Veröffentlichungstag der Anmeldung:
**12.11.2003 Patentblatt 2003/46**

(73) Patentinhaber: **BASF AKTIENGESELLSCHAFT
67056 Ludwigshafen (DE)**

(72) Erfinder:
- **SLANY, Michael
67281 Kirchheim (DE)**
- **SCHÄFER, Martin
67269 Grünstadt (DE)**
- **RÖPER, Michael
67157 Wachenheim (DE)**

(56) Entgegenhaltungen:

| | |
|---|---|
| **EP-A- 0 646 588** | **WO-A-00/14055** |
| **WO-A-02/00669** | **WO-A-96/19434** |
| **WO-A-99/24444** | **GB-A- 963 418** |
| **US-A- 3 496 204** | **US-A- 5 177 044** |
| **US-A- 5 179 225** | |

- **WELCHER R P ET AL: "4-PHOSPHORINANONES II" JOURNAL OF ORGANIC CHEMISTRY, AMERICAN CHEMICAL SOCIETY. EASTON, US, Bd. 27, Mai 1962 (1962-05), Seiten 1824-1827, XP001021630 ISSN: 0022-3263 in der Anmeldung erwähnt**
- **DATABASE WPI Section Ch, Week 198705 Derwent Publications Ltd., London, GB; Class E17, AN 1987-034019 XP002207576 & JP 61 291532 A (AGENCY OF IND SCIENCE & TECHNOL.)), 22. Dezember 1986 (1986-12-22)**
- **DATABASE CHEMICAL ABSTRACTS [Online] ASINGER, F. ET AL.: "Joint action of elementary sulfur and gaseous ammonia on ketones. LXIII. Action of sulfur and ammonia on cyclic ketones. 7. The action of sulfur and ammonia on 1-phenyl-2,2,6,6-tetramethylphosphorinan-4 -on" retrieved from STN Database accession no. 1969:11769 XP002207575 & MONATSHEFTE FÜR CHEMIE, Bd. 99, Nr. 5, 1968, Seiten 1695-1704,**
- **VEEN VAN DER L A ET AL: "NEW PHOSPHACYCLIC DIPHOSPHINES FOR RHODIUM-CATALYZED HYDROFORMAYLATION" ORGANOMETALLICS, WASHINGTON, DC, US, Bd. 18, Nr. 23, 8. November 1999 (1999-11-08), Seiten 4765-4777, XP000964505 ISSN: 0276-7333**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 360 005 B1

## Beschreibung

Die vorliegende Erfindung betrifft eine zur Herstellung eines Katalysatorsystems für die Carbonylierung un olefinisch ungesättigten Verbindungen (X) mittels CO und einer eine Hydroxylgruppe enthaltenden Verbindung (XI) geeignete Verbindung der Formel (I)

$$L^1 - X - L^2 \qquad (I)$$

mit

X: niedere Alkylengruppe mit 1 - 6 Kohlenstoffatomen zwischen dem beiden Resten $L_1$ und $L_2$, Arylengruppe oder Alkarylengruppe

$L^1$:

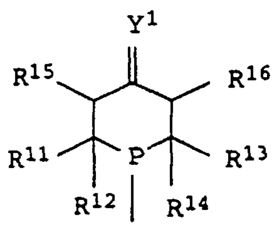

mit

| | |
|---|---|
| $Y^1$ | Sauerstoff, Schwefel oder N-$R^{17}$ |
| $R^{12}$, $R^{14}$,$R^{15}$, $R^{16}$, $R^{17}$ | unabhängig voneinander Wasser stoff, Alkyl oder Aryl |
| $R^{11}$, $R^{13}$ | unabhängig voneinander Alkyl oder Aryl |

$L^2$:

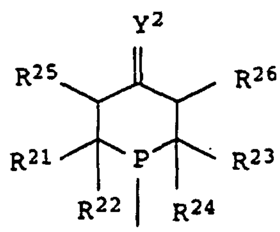

mit

| | |
|---|---|
| $Y^2$ | Sauerstoff, Schwefel oder N-$R^{27}$ |
| $R^{22}$, $R^{24}$, $R^{25}$, $R^{26}$, $R^{27}$ | unabhängig voneinander Wasser stoff, Alkyl oder Aryl |
| $R^{21}$, $R^{23}$ | unabhängig voneinander Alkyl oder Aryl |

wobei $L^1$ und $L^2$ gleich oder unterschiedlich sein können, und wobei die Gruppen X, $R^{11}$-$R^{17}$ und $R^{21}$-$R^{27}$ Substituiert sein können.

Ferner betrifft sie ein Verfahren zur Herstellung einer Verbindung der Formel (I), die Verwendung einer Verbindung der Formel (I), durch Umsetzung mit einer Verbindung der Formel (I) erhältliche als Katalysator geeignete Systeme (VI), sowie Verfahren zur Carbonylierung olefinisch ungesättigter Verbindungen in Gegenwart einer Verbindung der Formel (I) oder eines Systems der Formel (VI).

Verfahren zur katalytischen Carbonylierung, also der Umsetzung von olefinisch ungesättigten Verbindungen mit Kohlenmonoxid und einer eine Hydroxylgruppe enthaltenden Verbindung zu einer Säure oder deren Derivate in Gegenwart eines Katalysators, sind allgemein bekannt.

So können beispielsweise durch Carbonylierung von n-Pentensäure oder deren Derivate der Formel (XII) Adipinsäure oder deren Derivate erhalten werden, die in großem Umfang Anwendung bei der Herstellung technisch

bedeutender Polymere, insbesondere Polyamide, finden.

Verfahren zur Carbonylierung von n-Pentensäure oder deren Derivate der Formel (I) ist bekannt, beispielsweise aus GB-1497046, DE-A-2541640, US 4508660, EP-A-373579, US 4933483, EP-A-450577, US 4257973, WO 2000/14055, EP-A-577204, WO 2000/56695, EP-A-662467 oder WO 98/42717.

Bei der Carbonylierung von olefinisch ungesättigten Verbindungen (mit Ausnahme von Ethylen) werden üblicherweise geradkettige und verzweigte Produkte erhalten.

Im Falle der Carbonylierung von n-Pentensäure finden nur die geradkettigen Produkte in großem Umfang Anwendung, während die verzweigten Produkte keine oder nur eine mengenmäßig untergeordnete Bedeutung aufweisen.

Wünschenswert ist daher ein hohes n/i-Verhältnis bei gleichzeitig hoher Ausbeute. Unter dem n/i-Verhältnis wird das Verhältnis der Selektivität von geradkettigen Produkten zu der Selektivität der verzweigten Produkte verstanden. Die in diesem Zusammenhang im Stand der Technik genannte Linearität bezeichnet die Selektivität der geradkettigen Produkte. Das n/i-Verhältnis errechnet sich aus der Linearität gemäß der Gleichung

$$\text{n/i-Verhältnis} = \text{Linearität [\%]} / (100\ \% - \text{Linearität [\%]})$$

Unbefriedigend bei den genannten Verfahren ist das n/i-Verhältnis bei gleichzeitig hoher Ausbeute.
So wird gemäß US 4933483, Beispiel 6, ein n/i-Verhältnis von 24 (Linearität 96 %) bei einer Ausbeute von nur 70 % erreicht.

WO 98/42717 offenbart in Beispiel 7 eine Ausbeute von 84 % (Umsatz 100 %, Selektivität 84 %); allerdings beträgt das n/i-Verhältnis nur 5,25 (84 % lineares Produkt, Rest 16 % verzweigtes Produkt).

Der vorliegenden Erfindung lag die Aufgabe zugrunde, eine als Katalysator geeignete oder zur Herstellung eines als Katalysator geeigneten Systems geeignete Verbindung bereitzustellen, die die genannten Nachteile auf technisch einfache und wirtschaftliche Weise vermeidet.

Demgemäß wurde die eingangs definierte Verbindung der Formel (I), ein Verfahren zur Herstellung einer Verbindung der Formel (I), die Verwendung einer Verbindung der Formel (I), durch Umsetzung mit einer Verbindung der Formel (I) erhältliche als Katalysator geeignete Systeme (VI), sowie Verfahren zur Carbonylierung olefinisch ungesättigter Verbindungen in Gegenwart einer Verbindung der Formel (I) und eines Quelle eines Metall-Ions eines Metalls (VII) oder eines Systems der Formel (VI) gefunden.

Erfindungsgemäß weist die Verbindung der Formel (I) die Struktur

$$L^1 - X - L^2 \qquad (I)$$

auf.

Als X kommt eine niedere Alkylengruppe mit 1 bis 6, insbesondere 1 bis 4 Kohlenstoffatomen zwischen den beiden Resten $L^1$ und $L^2$, besonders bevorzugt Methylen, 1,2-Ethylen, 1,3-Propylen, 1,4-Butylen, eine Arylengruppe, vorzugsweise 1,2-Phenylen, 1,3-Phenylen, 1,4-Phenylen oder eine Alkarylengruppe, vorzugsweise 1,2-Benzyliden, 1,3-Benzyliden, 1,4-Benzyliden, 1,2-Xylyliden, 1,3-Xylyliden, 1,4-Xylyliden in Betracht.

In einer bevorzugten Ausführungsform kann man eine niedere Alkylengruppe, vorzugsweise eine solche mit 1 bis 6, insbesondere 1 bis 4 Kohlenstoffatomen zwischen den beiden Resten $L^1$ und $L^2$, besonders bevorzugt Methylen, 1,2-Ethylen, 1,3-Propylen, 1,4-Butylen einsetzen.

Die Arylengruppe, Alkylengruppe oder Alkarylengruppe kann Substituenten tragen, wie Alkylgruppen, beispielsweise Methyl, Ethyl, n-Propyl, i-Propyl, n-Butyl, i-Butyl, s-Butyl, t-Butyl, wie Arylgruppen, beispielsweise Phenyl, p-Tolyl, wie Halogene, beispielsweise Fluor, Chlor, Brom, Alkoxy, Aryloxy, vorzugsweise trägt sie keinen Substituenten.

Als $L^1$ kommt eine Struktur der Formel

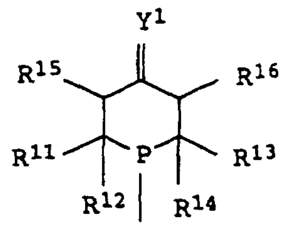

in Betracht.

In dieser Formel stellt $Y^1$ Sauerstoff, Schwefel oder N-$R^{17}$, vorzugsweise Sauerstoff, dar.

Als Gruppen $R^{11}$, $R^{12}$, $R^{13}$, $R^{14}$, $R^{15}$, $R^{16}$, $R^{17}$ kommen innenhalb der Definition des Anspruchs 1 unabhängig voneinander Wasserstoff, Alkyl, vorzugsweise Alkyl mit einem bis 6, insbesondere einem bis 4 Kohlenstoffatomen, oder Aryl, wie Phenyl, p-Tolyl in Betracht.

In einer bevorzugten Ausführungsform kann man als $R^{11}$, $R^{12}$, $R^{13}$, $R^{14}$, $R^{15}$, $R^{16}$, $R^{17}$ unabhängig voneinander einen Rest ausgewählt aus der Gruppe bestehend aus Wasserstoff, Methyl, Ethyl, n-Propyl, i-Propyl, n-Butyl, i-Butyl, s-Butyl, t-Butyl einsetzen.

Die Gruppen $R^{11}$, $R^{12}$, $R^{13}$, $R^{14}$, $R^{15}$, $R^{16}$, $R^{17}$ können unabhängig voneinander Substituenten tragen, wie Alkylgruppen, beispielsweise Methyl, Ethyl, n-Propyl, i-Propyl, n-Butyl, i-Butyl, s-Butyl, t-Butyl, wie Arylgruppen, beispielsweise Phenyl, p-Tolyl, wie Halogene, beispielsweise Fluor, Chlor, Brom, Alkoxy, Aryloxy, vorzugsweise tragen sie keinen Substituenten.

Als $L^2$ kommt eine Struktur der Formel

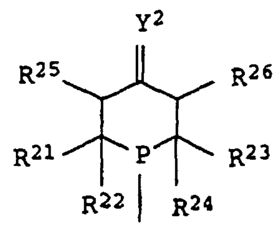

in Betracht.

In dieser Formel stellt $Y^2$ Sauerstoff, Schwefel oder N-$R^{27}$, vorzugsweise Sauerstoff, dar.

Als Gruppen $R^{21}$, $R^{22}$, $R^{23}$, $R^{24}$, $R^{25}$, $R^{26}$, $R^{27}$ kommen innenhalb der Definition des Anspuchs 1 unabhängig voneinander Wasserstoff, Alkyl, vorzugsweise Alkyl mit einem bis 6, insbesondere einem bis 4 Kohlenstoffatomen, oder Aryl, wie Phenyl, p-Tolyl in Betracht.

In einer bevorzugten Ausführungsform kann man als $R^{21}$, $R^{22}$, $R^{23}$, $R^{24}$, $R^{25}$, $R^{26}$, $R^{27}$ unabhängig voneinander einen Rest ausgewählt aus der Gruppe bestehend aus Wasserstoff, Methyl, Ethyl, n-Propyl, i-Propyl, n-Butyl, i-Butyl, s-Butyl, t-Butyl einsetzen.

Die Gruppen $R^{21}$, $R^{22}$, $R^{23}$, $R^{24}$, $R^{25}$, $R^{26}$, $R^{27}$ können unabhängig voneinander Substituenten tragen, wie Alkylgruppen, beispielsweise. Methyl, Ethyl, n-Propyl, i-Propyl, n-Butyl, i-Butyl, s-Butyl, t-Butyl, wie Arylgruppen, beispielsweise Phenyl, p-Tolyl, wie Halogene, beispielsweise Fluor, Chlor, Brom, Alkoxy, Aryloxy, vorzugsweise tragen sie keinen Substituenten.

Die Reste $L^1$ und $L^2$ können gleich oder unterschiedlich, vorzugsweise gleich sein.

Die Herstellung einer Verbindung der Formel (I) kann vorteilhaft erfolgen, indem man eine Verbindung der Formel (II)

$$H_2P - X - PH_2 \qquad (II)$$

mit einer Verbindung der Formel (IV)

$$(R^{11}\ R^{12}\ C) = (C\ R^{15})\ \text{-}(C{=}Y^{1})\ \text{-}\ (CR^{16}) = (CR^{13}\ R^{14}) \quad (IV)$$

und einer Verbindung der Formel (V)

$$(R^{21}\ R^{22}\ C) = (C\ R^{25})\ \text{-}(C{=}Y^{2})\ \text{-}\ (C\ R^{26}) = (C\ R^{23}\ R^{24}) \quad (V)$$

umsetzt, wobei $R^{11}$, $R^{12}$, $R^{13}$, $R^{14}$,$R^{15}$, $R^{16}$, $R^{21}$, $R^{22}$, $R^{23}$, $R^{24}$, $R^{25}$, $R^{26}$, $Y^1$, $Y^2$ und X die oben genannten Bedeutungen haben.

Als für die Herstellung vorteilhafte Verfahrensbedingungen können die Verfahrensbedingungen zur Herstellung von 4-Phosphorinanonen gemäß: Richard P. Welcher and Nancy E. Day, J. Org. Chem., 27 (1962) 1824-1827 angewandt werden.

Verbindungen der Formel (I) können als einzelne Substanz oder als Gemisch als Bestandteil eines als Katalysator geeigneten Systems gemäß Anspruch 8 verwendet werden.

So kommt die Verwendung eines Katalysators, der eine Verbindung der Formel (I) enthält, in einem Verfahren zur Carbonylierung von olefinisch ungesättigten Verbindungen (X) durch Umsetzung einer Verbindung der Formel (X) mit Kohlenmonoxid und einer eine Hydroxylgruppe enthaltenden Verbindung (XI) in Betracht.

Ein als Katalysator geeignetes System (VI) kann vorteilhaft erhältlich sein durch Umsetzung von

a) einer Quelle für ein Metall-Ion eines Metalls (VII) der VIII. Nebengruppe des Periodensystems der Elemente mit

b) einer Verbindung der Formel (I) wie oben beschrieben.

Als Metall (VII) kommt ein Metall der VIII. Nebengruppe des Periodensystems, wie Eisen, Cobalt, Nickel, Ruthenium, Rhodium, Palladium, Osmium, Iridium, Platin, vorzugsweise Palladium, Platin, Rhodium, Iridium, insbesondere Palladium, in Betracht, sowie deren Gemische.

Als Quelle für ein Metall-Ion eines solchen Metalls kann man vorteilhaft Salze solcher Metalle mit, oder Verbindungen, in denen ein solches Metall schwach koordinativ mit ein Anion verbunden ist, die sich ableiten von Mineralsäuren, wie Salpetersäure, Schwefelsäure, Phosphorsäure, Carbonsäuren, vorteilhaft $C_1$-$C_{12}$-Carbonsäure, vorzugsweise Essigsäure, Propionsäure, Buttersäure, Sulfonsäure, wie Methansulfonsäure, Chlorsulfonsäure, Fluorsulfonsäure, Trifluormethansulfonsäure, Benzolsulfonsäure, Naphthalinsulfonsäure, Toluolsulfonsäure, insbesondere p-Toluolsulfonsäure, t-Butylsulfonsäure, 2-Hydroxypropansulfonsäure, sulfonierte Ionentauscher, Halogenpersäuren, wie Perchlorsäure, Perfluorierte Carbonsäuren, wie Trifluoressigsäure, Nonafluorbutansulfonsäure, Trichloressigsäure, Phosphonsäuren, wie Benzolphosphonsäure, Säuren, die sich ableiten aus der Wechselwirkung von Lewis-Säuren mit Broensted-Säuren, Anionen, wie Tetraphenylborat und Derivate hiervon, oder deren Gemische einsetzen.

Ebenso können Verbindungen vorteilhaft eingesetzt werden, in denen ein solches Metall nullwertig mit leicht abtrennbaren Liganden vorliegt, wie beispielweise Tris(dibenzylidenaceton) palladium, Tetrakis(triphenylphosphan) palladium, Bis(trio-tolylphosphan)palladium.

Vorteilhaft kommen dabei solche Verbindungen der Formel (I) in Betracht, die beider Umsetzung mit einem Metall (VII) oder Metall-Ion (VII) chelatisierende Wirkung zeigen.

Das molare Verhältnis von Verbindung (I) zu Metall (VII) kann in einem weiten Bereich gewählt werden. Vorteilhaft kommt ein Verhältnis im Bereich von 0,5 bis 50, vorzugsweise 0,5 bis 20 besonders bevorzugt 0,5 bis 10, insbesondere 1 bis 5 mol/mol in Betracht.

In einer bevorzugten Ausführungsform ist das Katalysatorsystem erhältlich in Gegenwart einer Anionenquelle (IX).

Als Anionenquelle können Verbindungen eingesetzt werden, die das Anion bereits enthalten, wie Salze, oder Verbindungen, die durch chemische Reaktion, wie heterolytische Bindungsspaltung, ein Anion freisetzen können, eingesetzt werden.

Geeignete Anionenquellen sind beispielsweise aus EP-A-495 547 bekannt.

Als Anionenquelle (IX) kann man vorteilhaft Verbindungen einsetzen, die in der Lage sind, unter Abspaltung eines $H^+$-Ions ein Anion zur Verfügung zu stellen, wie Salpetersäure, Schwefelsäure, Phosphorsäure, Carbonsäuren, vorteilhaft $C_1$-$C_{20}$-Carbonsäure, vorzugsweise Essigsäure, Propionsäure, 2,4,6-Trimethylbenzoesäure, 2,6-Dichlorbenzoesäure, 9-Anthracencarbonsäure, Pivalinsäure, 1,2,3-Benzoltricarbonsäure, 1,2,3-Benzoltricarbonsäure-1,3-Diester, 2-Ethoxy-1-naphthalincarbonsäure, 2,6-Dimethoxybenzoesäure, 5-Cyanvaleriansäure, Sulfonsäure, wie Methansulfonsäure, Chlorsulfonsäure, Fluorsulfonsäure, Trifluormethansulfonsäure, Benzolsulfonsäure, Naphthalinsulfonsäure, Toluolsulfonsäure, insbesondere p-Toluolsulfonsäure, t-Butylsulfonsäure, 2-Hydroxypropansulfonsäure, sulfonierte Ionentauscher, Halogenpersäuren, wie Perchlorsäure, Perfluorierte Carbonsäuren, wie Trifluoressigsäure, Nonafluorbutansulfonsäure, Trichloressigsäure, Phosphonsäuren, wie Benzolphosphonsäure, Säuren, die sich ableiten aus der Reaktion von Lewis-Säuren, wie $BF_3$, $PF_5$, $AsF_5$, $SbF_5$, $TaF_5$ oder $NbF_5$, mit einer Broensted Säure, wie HF (beispielsweise Fluorosilikatsäure, $HBF_4$, $HPF_6$, $HSbF_6$, Tetraphenylborsäure und Derivate hiervon) oder deren Gemische.

Unter den Verbindungen (IX), die in der Lage ist, unter Abspaltung eines $H^+$-Ions ein Anion zur Verfügung zu stellen, sind diejenigen bevorzugt, die einen $pK_a$-Wert von höchstens 3,5, insbesondere höchstens 2 aufweisen.

Das molare Verhältnis von Verbindung (IX) zu Metall (VII) ist an sich nicht kritisch. Vorteilhaft kann das molare Verhältnis von Verbindung (IX) zu Metall (VII) im Bereich von 0,5 bis 100, vorzugsweise 1 bis 20 mol/mol liegen.

Erfindungsgemäß kann in einem Verfahren zur Carbonylierung von olefinisch ungesättigten Verbindungen (X) durch Umsetzung einer Verbindung der Formel (X) mit Kohlenmonoxid und einer eine Hydroxylgruppe enthaltenden Verbindung (XI) in Gegenwart eines Katalysatorsystems ein Katalysator eingesetzt werden, der ein System (VI) enthält, vorzugsweise aus ihm besteht.

Als Verbindung (X) können grundsätzlich intern und terminal olefinisch ungesättigte Verbindungen ohne Beschränkung eingesetzt werden. Verbindung (X) kann einfach oder mehrfach, wie zweifach oder dreifach, vorzugsweise einfach, olefinisch ungesättigt sein.

In einer bevorzugten Ausführungsform kann als olefinisch ungesättigte Verbindung vorteilhaft ein substituiertes oder unsubstituiertes Alken oder Cycloalken, vorzugsweise mit 2 bis 30, insbesondere 2 bis 20, besonders bevorzugt 2 bis 10 Kohlenstoffatomen im Molekül sein. Das Alken oder Cycloalken kann substituiert sein beispielsweise mit einem oder mehreren Halogenatomen, Cyano-, Ester-, Carboxy-, Amino-, Amido-, Nitril-, Alkoxy-, Aryloder Thioalkoxy-Gruppen. Beispiele für olefinisch ungesättigte Verbindungen (X) sind Ethen, Propen, 1-Buten, 2-Buten, Isobuten, die isomeren Pentene, Hexene, Octene, wie 1-Octen, 2,4,4-Trimethyl-1-penten, 2,4,4-Trimethyl-2-penten, und Dodecene, 1,5-Cyclooctadien, Cyclododecen, Acrylsäure, Methylacrylat, Ethylacrylat, Acrylnitril, Acrylamid, N,N-Dialkylacrylamid, Acrylaldehyd, Methylmethacrylat, die isomeren Pentensäuren, die isomeren Pentensäuremethylester, die isomeren Pentennitrile, Vinylchlorid, Ethylvinylketon, Allylchlorid, Methylallylether, Styrol.

In einer weiteren bevorzugten Ausführungsform kommt als Verbindung (X) eine gegebenenfalls substituierte olefinisch ungesättigte Verbindung mit mindestens einer terminalen olefinischen Bindung, insbesondere ein gegebenenfalls substituiertes alpha-Olefin, in Betracht. Bevorzugte gegebenenfalls substituierte olefinisch ungesättigte Verbindungen können dargestellt werden durch die Formel (XIII)

$$H_2C = (C\ Y^3\ Y^4) \qquad (XIII)$$

In der $Y^3$ für Wasserstoff oder eine Hydrocarbylgruppe und $Y^4$ für Wasserstoff oder einen elektronenziehenden oder elektronenschiebenden Substituenten, wie Carboxy, Nitril, Formyl, Amino, Halogen oder Substituenten der Formel $Y^5$-$R^{41}$ stehen, wobei $Y^5$ für eine Einfachbindung oder die Funktionalität -CO-, -COO-, -OOC-, -NH-, -CONH-, -NHCO-, -O- oder -S- steht und $R^{41}$ für eine gegebenenfalls substituierte Alkyl-, Alkenyl-, Cycloalkyl-, Cycloalkenyl-, Aryloder heterocyclische Gruppe. Als Alkylgruppe kommt vorteilhaft eine $C_1$- bis $C_{10}$-Alkylgruppe, insbesondere eine $C_1$ bis $C_6$-Alkylgruppe, wie Methyl, Ethyl, n-Propyl, i-Propyl, n-Butyl, i-Butyl, s-Butyl, t-Butyl und n-Hexyl, in Betracht. Als Cycloalkylgruppe kann vorteilhaft eine $C_3$- bis $C_6$-Cycloalkylgruppe, wie Cyclopentyl oder Cyclohexyl, eingesetzt werden. Die Alkenyl- und Cycloalkenyl-Gruppen können vorteilhaft die gleiche Zahl an Kohlenstoffatomen und eine Kohlenstoff-Kohlenstoff-Doppelbindung in irgendeiner Position aufweisen. Die Arylgruppe kann vorteilhaft eine Phenyl- oder Naphthylgruppe sein. Die heterocyclische Gruppe sollte vorzugsweise 3 bis 12 Atome einschließlich 1, 2 oder 3 Heteroatome, wie Sauerstoff, Schwefel oder Stickstoff, aufweisen.

In einer weiteren bevorzugten Ausführungsform kann man als Verbindung (X) Nukleophile mit einem beweglichen Wasserstoff einsetzen, einschließlich Alkensäure-Derivate, wie Alkensäuren, Alkensäureanhydride, Alkensäureamide, Alkensäurenitrile oder Alkensäureester. Die Säuregruppe kann sich in direkter Nachbarschaft zur olefinischen Doppelbindung befinden, beispielsweise ein 2-Alkensäure-Derivat darstellen. Die Alkenylgruppe der Alkensäure kann substituiert oder unsubstituiert, vorzugsweise unsubstituiert sein, wie Vinyl, 1-Propenyl, 1-Butenyl, 1-Pentenyl oder 1-Hexenyl, und vorteilhaft 2 bis 12 Kohlenstoffatome aufweisen. In Betracht kommen beispielsweise Acrylsäure, Methacrylsäure, 2-Butensäure, 2-Pentensäure, Acrylnitril, Methyacrylnitril, 2-Butennitril, 2-Pentennitril, Acrylamid, Methacrylamid, 2-Butenamid, 2-Pentenamid, N-substituiertes Acrylamid, N-substituiertes Methacrylamid, N-substituiertes 2-Butenamid, N-substituiertes 2-Pentenamid oder Ester der genannten Alkensäuren. Die N-Substituenten der Amidgruppe und die O-Substituenten der Estergruppe können aliphatisch, cycloaliphatisch oder aromatisch, unsubstituiert oder substituiert, sein und vorzugsweise 1 bis 10 Kohlenstoffatome aufweisen. Beispiele hierfür sind Methylacrylat, Ethylacrylat, Phenylacrylat, n-Propylacrylat, i-Propylacrylat, n-Butylacrylat, i-Butylacrylat, t-Butylacrylat, s-Butylacrylat,

die entsprechenden Methacrylate, 2-Butenate, 2-Pentenate, N,N-Dimethylacrylamid und die entsprechenden Methacrylamide, 2-Butenamide und 2-Pentenamide.

In einer weiteren bevorzugten Ausführungsform kann man als Verbindung (X) ein unsubstituiertes alpha-Olefin in Gegenwart eines Nukleophils mit einem beweglichen Wasserstoff carbonylieren unter Erhalt eines Esters oder einer anderen Carbonylverbindung.

In einer weiteren bevorzugten Ausführungsform kann man als Verbindung (X) n-Pentensäure oder deren Derivate der Formel (XII)

$$C_4H_7 - R^{31} \qquad \text{(XII)}$$

einsetzen, wobei im Sinne der vorliegenden Erfindung hierunter auch Gemische solcher Verbindungen verstanden werden.

Als Rest $R^{31}$ kommt -CN oder $COOR^{32}$ in Betracht, wobei $R^{32}$ Wasserstoff, Alkyl, oder Aryl, vorteilhaft Wasserstoff oder Alkyl, vorzugsweise Wasserstoff, Methyl, Ethyl, n-Propyl, i-Propyl, n-Butyl, i-Butyl, s-Butyl, t-Butyl, insbesondere Wasserstoff, Methyl, Ethyl, besonders bevorzugt Wasserstoff oder Methyl sein kann.

Falls $R^{32}$ eine Alkyl- oder Aryl-Gruppe ist, kann diese Substituenten, wie funktionelle Gruppen oder weitere Alkyl- oder Arylgruppen tragen. Vorzugsweise trägt $R^2$ im Falle einer Alkyloder Aryl-Gruppe keine Substituenten.

Als n-Pentensäure oder deren Derivate der Formel (XII) kommen grundsätzlich alle Isomeren, wie cis-2-, trans-2-, cis-3-, trans-3- und 4-Isomer, sowie deren Gemische in Betracht. Solche Gemische können den gleichen oder unterschiedliche Reste $R^{31}$ aufweisen. Bevorzugt sind solche Gemische, die den gleichen Rest $R^{31}$ aufweisen.

Vorteilhaft kommt der Einsatz von cis-2-, trans-2-, cis-3-, trans-3- oder 4-Pentennitril sowie deren Gemische in Betracht. Bevorzugt sind dabei solche Gemische, die mindestens 80 Gew.-% 3-Pentennitril, also die Summe aus cis-3-Pentennitril und trans-3-Pentennitril, enthalten.

In einer weiteren vorteilhaften Ausführungsform kommt der Einsatz von cis-2-, trans-2-, cis-3-, trans-3- oder 4-Pentensäure sowie deren Gemische in Betracht. Bevorzugt sind dabei solche Gemische, die mindestens 80 Gew.-% 3-Pentensäure, also die Summe aus cis-3-Pentensäure und trans-3-Pentensäure, enthalten.

In einer weiteren vorteilhaften Ausführungsform kommt der Einsatz von cis-2-, trans-2-, cis-3-, trans-3- oder 4-Pentensäuremethylester sowie deren Gemische in Betracht. Bevorzugt sind dabei solche Gemische, die mindestens 80 Gew.-% 3-Pentensäuremethylester, also die Summe aus cis-3-Pentensäuremethylester und trans-3-Pentensäuremethylester, enthalten.

Pentensäure und deren Derivate gemäß Formel (XII) können nach an sich bekannten Verfahren, beispielsweise durch Addition von Kohlenmonoxid und einer eine Hydroxylgruppe enthaltenden Verbindung oder von Cyanwasserstoff an Butadien in Gegenwart eines Katalysators, erhalten werden.

Das molare Verhältnis von Metall (VII) zu Verbindung (X) ist an sich nicht kritisch. Als vorteilhaft hat sich ein molares Verhältnis von Metall (VII) zu Verbindung (X) im Bereich von $10^{-7}$:1 bis $10^{-1}$:1, vorzugsweise $10^{-6}$:1 bis $10^{-2}$:1 erwiesen.

Erfindungsgemäß setzt man Verbindung der Formel (X) mit Kohlenmonoxid um. Dabei kann Kohlenmonoxid als reine Verbindung oder in Gegenwart von Gasen, die das erfindungsgemäße Verfahren im wesentlichen nicht nachteilig beeinflussen, insbesondere sich inert verhalten, eingesetzt werden. Als solche Inerte kommen beispielsweise Stickstoff, Wasserstoff, Kohlendioxid, Methan und die Edelgase, wie Argon, in Betracht.

Vorteilhaft kann das molare Verhältnis von Verbindung (X) zu Kohlenmonoxid mindestens 1:1, vorzugsweise mindestens 3:1, insbesondere mindestens 5:1 betragen, vorzugsweise im Bereich von 5:1 bis 50:1, besonders bevorzugt im Bereich von 7:1 bis 15:1 liegen. Führt man das erfindungsgemäße Verfahren bei molaren Verhältnissen von Verbindung (I) zu Kohlenmonoxid kleiner als 5:1, insbesondere kleiner als 3:1, speziell kleiner 1:1 durch, so kann dies zu einer raschen Verschlechterung der Eigenschaften des Katalysatorsystems führen.

Erfindungsgemäß setzt man Verbindung der Formel (X) mit einer eine Hydroxyl-Gruppe enthaltenden Verbindung (XI) um. Im Sinne der vorliegenden Erfindung werden unter Verbindung (XI) einzelne Verbindungen (XI) wie auch Gemische verschiedener solcher Verbindungen verstanden.

Die Art von Verbindung (XI) bestimmt mindestens teilweise das Endprodukt des vorliegenden Verfahrens. Setzt man Wasser als Verbindung (XI) ein, so erhält man die entsprechende Säure, während bei Einsatz eines Alkohols, wie eines Alkanols, der entsprechende Ester erhalten wird. Als Alkohol kommen primäre, sekundäre oder tertiäre, vorzugsweise primäre, Alkohole in Betracht, vorteilhaft $C_1$-$C_{30}$-Alkanole, die gegebenenfalls Substituenten tragen können, wie eine oder mehrere Halogen-, Nitril-, Carbonyl-, Alkoxy- oder Aryl-Gruppen. Vorteilhaft kommen als Alkanol Methanol, Ethanol, n-Propanol, i-Propanol, n-Butanol, i-Butanol, s-Butanol, t-Butanol, n-Hexanol, n-Octanol, i-Octanol, 2-Ethylhexanol, Cyclohexanol, Benzylalkohol, Phenylethylalkohol, Ethylenglykol, 1,2-Propandiol, 1,3-Propandiol, Neopentylglykol, Trimethylolpropan, Pentaerythriol, bevorzugt Methanol, Ethanol, n-Propanol, i-Propanol, n-Butanol, i-Butanol, s-Butanol, t-Butanol, besonders bevorzugt Methanol oder Ethanol, insbesondere Methanol in Betracht.

Das molare Verhältnis von Verbindung (X) zu Verbindung (XI) ist an sich nicht kritisch und kann in einem weiten Bereich liegen, vorteilhaft im Bereich von 0,001:1 bis 100:1 mol/mol.

Das Katalysatorsystem kann vor dem Einsatz in dem erfindungsgemäßen Verfahren oder in dem erfindungsgemäßen Verfahren selbst hergestellt werden.

Stellt man das Katalysatorsystem in dem erfindungsgemäßen Verfahren selbst her, so hat sich der Einsatz von solchen Verbindungen von Metall (III) als vorteilhaft erwiesen, die in dem Reaktionsgemisch soweit löslich sind, daß sie mit den anderen Komponenten ein aktives Katalysatorsystem bilden können.

Das im erfindungsgemäßen Verfahren eingesetzte Katalysatorsystem kann in homogener oder heterogener, vorzugsweise homogener Phase eingesetzt werden.

Das Katalysatorsystem kann vorteilhaft in flüssiger Phase erhalten werden. Die flüssige Phase kann dabei durch eine oder mehrere der Komponenten, aus denen das Katalysatorsystem erhältlich ist oder erhalten wurde, gebildet werden. Ebenso ist es möglich, die flüssige Phase durch ein anorganisches oder organisches, vorzugsweise organisches flüssiges Verdünnungsmittel bereitzustellen.

Als flüssiges Verdünnungsmittel kommen vorteilhaft aprotische flüssige Verdünnungsmittel in Betracht, wie Ether, beispielsweise Diethylether, Dimethylether, Dimethylether von Ethylenglykol, Dimetyhlether von Diethylenglykol, Tetrahydrofuran, Polyether, funktionalisierte Polyether, Anisol, 2,5,8-Trioxanonan, Diisopropylether, Diphenylether, wie Aromaten, einschließlich halogenierter Aromaten, beispielsweise Benzol, Toluol, o-Xylol, m-Xylol, p-Xylol, Chlorbenzol, o-Dichlorbenzol, m-Dichlorbenzol, p-Dichlorbenzol, wie Alkane, einschließlich halogenierter Alkane, beispielsweise Hexan, Heptan, 2,2,3-Trimethylpentan, Methylendichlorid, Tetrachlormethan, wie Nitrile, beispielsweise Benzonitril, Acetonitril, wie Ester, beispielsweise Methylbenzoat, Methylacetat, Dimethylphthalat, Butyrolacton, wie Sulfone, beispielsweise Diethylsulfon, Diisopropylsulfon, Tetrahydrothiophen-1,1-dioxid ("Sulfolan"), 2-Methyl-sulfolan, 3-Methylsulfolan, 2-Methyl-4-butyl-sulfolan, wie Sulfoxide, beispielsweise Dimethylsulfoxid, wie Amide, einschließlich halogenierter Amide, beispielsweise Dimethylformamid, Dimethylacetamid, N-Methylpyrrolidon, wie Ketone, beispielsweise Aceton, Methylethylketon, Methyl-isobutyl-keton, sowie deren Gemische.

Besonders bevorzugt sind solche flüssigen Verdünnungsmittel, deren Siedepunkt höher ist als der Siedepunkt des nach dem erfindungsgemäßen Verfahren erhaltenen jeweiligen Produkts. Hierdurch kann die Abtrennung des Produkts von der restlichen Reaktionsmischung, beispielsweise durch Destillation, erleichtert werden.

Das erfindungsgemäße Verfahren kann vorteilhaft bei einer Temperatur im Bereich von 20 bis 250°C, vorzugsweise 40 bis 200°C, besonders bevorzugt 70 bis 170°C, insbesondere 80 bis 140°C, durchgeführt werden.

Das erfindungsgemäße Verfahren kann vorteilhaft bei einem Gesamtdruck von $1*10^5$ bis $200*10^5$ Pa, vorzugsweise $5*10^5$ bis $70*10^5$ Pa, insbesondere $6*10^5$ bis $20*10^5$ Pa, durchgeführt werden.

Das erfindungsgemäße Verfahren kann kontinuierlich, diskontinuierlich oder semi-kontinuierlich durchgeführt werden.

Das Verfahrensprodukt kann von den anderen Komponenten nach an sich bekannten Verfahren, wie Extraktion oder Destillation, abgetrennt werden.

Durch das hohe n/i-Verhältnis des erfindungsgemäßen Verfahrens ist der nachfolgende Reinigungsaufwand deutlich reduziert, da weniger unerwünschte Nebenprodukte erhalten werden.

Ein weiterer Vorteil der erfindungsgemäßen Verfahrens besteht darin, daß die restlichen Komponenten, die das Katalysatorsystem enthalten, in das erfindungsgemäße Verfahren zurückgeführt werden können. Dabei kann gewünschtenfalls neuer Katalysator zugesetzt werden.

Beispiele

Herstellung von Verbindungen der Formel (I)

Beispiel 1

14,6 g (0,106 mol) 2,6-Dimethyl-2,5-heptadien-4-on ("Phoron") und 5,0 g (0,053 mol) 1,2-Bisphosphinoethan wurden in einem Schlenkrohr zusammengegeben und für 20 Stunden bei 120°C gerührt. Dabei bildete sich ein hellgelber Feststoff, der zweimal mit je 20 ml Pentan gewaschen und anschließend im Vakuum getrocknet wurde unter Erhalt eines weißen Pulvers. Die Charakterisierung des Produkts erfolgte mit Hilfe von 31P-NMR-Spektroskopie, Elementaranalyse und GC/MS.
Ausbeute: 18,2 g entsprechend 93 % der Theorie

Beispiel 2

Es wurde verfahren wie in Beispiel 1, jedoch wurde statt 1,2-Bisphosphinoethan 5,7 g (0,053 mol) 1,3-Bisphosphinopropan eingesetzt.
Ausbeute: 18,7 g entsprechend 92 % der Theorie

Beispiel 3

Es wurde verfahren wie in Beispiel 1, jedoch wurde statt 1,2-Bisphosphinoethan 6,5 g (0,053 mol) 1,3-Bisphosphinopropan eingesetzt.
Ausbeute: 19,9 g entsprechend 94 % der Theorie

Beispiel 4

Es wurde verfahren wie in Beispiel 1, jedoch wurde statt 1,2-Bisphosphinoethan 7,5 g (0,053 mol) 1,2-Bisphosphinobenzol eingesetzt.
Ausbeute: 19,9 g entsprechend 86 % der Theorie

Beispiel 5

Es wurde verfahren wie in Beispiel 1, jedoch wurden 2 g (0,021 mol) 1,2-Bisphosphinoethan und 10 g (0,0427 mol) Dibenzylidenaceton eingesetzt.
Ausbeute: 11,0 g entsprechend 92 % der Theorie

Beispiel 6

Es wurde verfahren wie in Beispiel 5, jedoch wurden statt 1,2-Bisphosphinoethan 2,3 g (0,021 mol) 1,3-Bisphosphinopropan eingesetzt.
Ausbeute: 11,1 g entsprechend 90 % der Theorie

Herstellung von Systemen der Formel (VI)

Beispiel 7

8,5 g (0,023 mol) 1,2-Bis(4-phosphorinon)ethan gemäß Beispiel 1 wurde in 50 ml Aceton gelöst und zu einer Lösung von Palladium-di-acetat in 50 ml Aceton langsam zugetropft. Sofort fiel ein gelber Feststoff aus, der mittels einer Fritte abgetrennt, zweimal mit je 20 ml Toluol gewaschen und im Vakuum getrocknet wurde. Ausbeute: 13,2 g entsprechend 96 % der Theorie.

Beispiel 8

Es wurde verfahren wie in Beispiel 7, jedoch wurde statt 1,2-Bis(4-phosphorinon)ethan 8,8 g (0,023 mol) 1,2-Bis(4-phosphorinon)propan gemäß Beispiel 2 eingesetzt.
Ausbeute: 13,5 g entsprechend 96 % der Theorie

Beispiel 9

Es wurde verfahren wie in Beispiel 7, jedoch wurde statt 1,2-Bis(4-phosphorinon)ethan 9,2 g (0,023 mol) 1,2-Bis(4-phosphorinon)butan gemäß Beispiel 3 eingesetzt.
Ausbeute: 13,8 g entsprechend 96 % der Theorie

Carbonylierung von Ethen

Beispiel 10

28 mg (0,12 mmol) Palladium-di-acetat, 81 mg (0,22 mmol) 1,2-Bis(4-phosphorinon)ethan gemäß Beispiel 1, 94 mg (0,5 mmol) p-Toloulsulfonsäure und 150 ml n-Butanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 90°C erhitzt und der Gesamtdruck auf 7 bar ($7*10^5$ Pa) bei einem molaren Verhältnis von Kohlenmonoxid zu Ethen von 1:1 eingestellt. Nach einer Stunde wurde der Autoklav abgekühlt entspannt und der Flüssigaustrag gaschromatographisch untersucht. Es wurde keine Palladiumabscheidung beobachtet.

Die Ergebnisse sind in Tabelle 1 zusammengefaßt.

Beispiel 11

28 mg (0,12 mmol) Palladium-di-acetat, 81 mg (0,22 mmol) 1,2-Bis(4-phosphorinon)ethan gemäß Beispiel 1, 60 mg (0,6 mmol) Methansulfonsäure und 150 ml n-Butanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 90°C erhitzt und der Gesamtdruck auf 7 bar ($7*10^5$ Pa) bei einem molaren Verhältnis von Kohlenmonoxid zu Ethen von 1:1 eingestellt. Nach einer Stunde wurde der Autoklav abgekühlt entspannt und der Flüssigaustrag gaschromatographisch untersucht. Es wurde keine Palladiumabscheidung beobachtet.
Die Ergebnisse sind in Tabelle 1 zusammengefaßt.

Beispiel 12

28 mg (0,12 mmol) Palladium-di-acetat, 81 mg (0,22 mmol) 1,2-Bis(4-phosphorinon)ethan gemäß Beispiel 1, 410 mg (23 mmol) Wasser und 150 ml n-Butanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 140°C erhitzt und der Gesamtdruck auf 7 bar ($7*10^5$ Pa) bei einem molaren Verhältnis von Kohlenmonoxid zu Ethen von 1:1 eingestellt. Nach einer Stunde wurde der Autoklav abgekühlt entspannt und der Flüssigaustrag gaschromatographisch untersucht. Es wurde keine Palladiumabscheidung beobachtet.
Die Ergebnisse sind in Tabelle 1 zusammengefaßt.

Beispiel 13

28 mg (0,12 mmol) Palladium-di-acetat, 81 mg (0,22 mmol) 1,2-Bis(4-phosphorinon)ethan gemäß Beispiel 1, 245 mg (0,1 mmol) 9-Anthracencarbonsäure und 150 ml n-Butanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 140°C erhitzt und der Gesamtdruck auf 7 bar ($7*10^5$ Pa) bei einem molaren Verhältnis von Kohlenmonoxid zu Ethen von 1:1 eingestellt. Nach einer Stunde wurde der Autoklav abgekühlt entspannt und der Flüssigaustrag gaschromatographisch untersucht. Es wurde keine Palladiumabscheidung beobachtet.
Die Ergebnisse sind in Tabelle 1 zusammengefaßt.

Beispiel 14

36 mg (0,06 mmol) System Gemäß Beispiel 7, 30 mg (0,3 mmol) Methansulfonsäure und 150 ml n-Butanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 90°C erhitzt und der Gesamtdruck auf 7 bar ($7*10^5$ Pa) bei einem molaren Verhältnis von Kohlenmonoxid zu Ethen von 1:1 eingestellt. Nach einer Stunde wurde der Autoklav abgekühlt entspannt und der Flüssigaustrag gaschromatographisch untersucht. Es wurde keine Palladiumabscheidung beobachtet.
Die Ergebnisse sind in Tabelle 1 zusammengefaßt.

Tabelle 1

| Beispiel | Umsatz n-Butanol [%] | TOF [$hr^{-1}$] | Selektivität Propionsäureester [%] |
|---|---|---|---|
| 10 | 80 | 10950 | >99 |
| 11 | 95 | 12900 | >99 |
| 12 | 50 | 6840 | >99 |
| 13 | 45 | 6150 | >99 |
| 14 | 98 | 26700 | >99 |
| TOF: Turn-over-frequency (mol Produkt / mol Katalysator/ Stunde) | | | |

Carbonylierung von 3-Pentennitril

Beispiel 15

70 mg (0,31 mmol) Palladium-di-acetat, 230 mg (0,62 mmol) 1,2-Bis(4-phosphorinon)ethan gemäß Beispiel 1, 590 mg (3,1 mmol) p-Toloulsulfonsäure, 80 ml (830 mmol) 3-Pentennitril und 34 ml Methanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 90°C erhitzt und der Gesamtdruck auf 7 bar ($7*10^5$ Pa) eingestellt. Nach einer Stunde wurde der Autoklav abgekühlt entspannt und der Flüssigaustrag gaschromatographisch untersucht. Es wurde keine Palladiumabscheidung beobachtet.
Die Ergebnisse sind in Tabelle 2 zusammengefaßt.

Beispiel 16

70 mg (0,31 mmol) Palladium-di-acetat, 240 mg (0,62 mmol) 1,3-Bis(4-phosphorinon)propan gemäß Beispiel 2, 590 mg (3,1 mmol) p-Toloulsulfonsäure, 80 ml (830 mmol) 3-Pentennitril und 34 ml Methanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 90°C erhitzt und der Gesamtdruck auf 7 bar ($7*10^5$ Pa) eingestellt. Nach einer Stunde wurde der Autoklav abgekühlt entspannt und der Flüssigaustrag gaschromatographisch untersucht. Es wurde keine Palladiumabscheidung beobachtet.
Die Ergebnisse sind in Tabelle 2 zusammengefaßt.

Beispiel 17

123 mg (0,2 mmol) System (VI) gemäß Beispiel 8, 380 mg (2,0 mmol) p-Toloulsulfonsäure, 80 ml (830 mmol) 3-Pentennitril und 34 ml Methanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 90°C erhitzt und der Gesamtdruck auf 7 bar ($7*10^5$ Pa) eingestellt. Nach einer Stunde wurde der Autoklav abgekühlt entspannt und der Flüssigaustrag gaschromatographisch untersucht. Es wurde keine Palladiumabscheidung beobachtet.
Die Ergebnisse sind in Tabelle 2 zusammengefaßt.

Tabelle 2

| Bsp. | Umsatz an 3-PN [%] | TOF [$hr^{-1}$] | Carbonylierungs-Selektivität [%] | n-Selektivität [%] |
|---|---|---|---|---|
| 15 | 70 | 1705 | 91 | 80,5 |
| 16 | 94 | 2500 | >99 | 97,2 |
| 17 | 95 | 3900 | >99 | 98,3 |

3-PN: 3-Pentennitril
TOF: Turn-over-frequency (mol Produkt / mol Katalysator/ Stunde)

Carbonylierung von 3-Pentensäuremethylester

Beispiel 18

70 mg (0,31 mmol) Palladium-di-acetat, 230 mg (0,62 mmol) 1,2-Bis(4-phosphorinon)ethan gemäß Beispiel 1, 590 mg (3,1 mmol) p-Toloulsulfonsäure, 102 ml (840 mmol) 3-Pentensäuremethylester und 34 ml Methanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 90°C erhitzt und der Gesamtdruck auf 7 bar ($7*10^5$ Pa) eingestellt. Nach einer Stunde wurde der Autoklav abgekühlt entspannt und der Flüssigaustrag gaschromatographisch untersucht. Es wurde keine Palladiumabscheidung beobachtet.
Die Ergebnisse sind in Tabelle 3 zusammengefaßt.

Beispiel 19

70 mg (0,31 mmol) Palladium-di-acetat, 240 mg (0,62 mmol) 1,3-Bis(4-phosphorinon)propan gemäß Beispiel 2, 590 mg (3,1 mmol) p-Toloulsulfonsäure, 102 ml (840 mmol) 3-Pentensäuremethylester und 34 ml Methanol wurden

in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 90°C erhitzt und der Gesamtdruck auf 7 bar ($7*10^5$ Pa) eingestellt. Nach einer Stunde wurde der Autoklav abgekühlt entspannt und der Flüssigaustrag gaschromatographisch untersucht. Es wurde keine Palladiumabscheidung beobachtet.

Die Ergebnisse sind in Tabelle 3 zusammengefaßt.

Beispiel 20

123 mg (0,2 mmol) System (VI) gemäß Beispiel 8, 380 mg (2,0 mmol) p-Toloulsulfonsäure, 102 ml (840 mmol) 3-Pentensäuremethylester und 34 ml Methanol wurden in einen 400 ml-Glasautoklaven mit Begasungsrührer gegeben. Nach dem Verschließen wurde bis zu einem Druck von 4 bar ($4*10^5$ Pa) Kohlenmonoxid aufgepreßt. Der Autoklav wurde auf 90°C erhitzt und der Gesamtdruck auf 7 bar ($7*10^5$ Pa) eingestellt. Nach einer Stunde wurde der Autoklav abgekühlt entspannt und der Flüssigaustrag gaschromatographisch untersucht. Es wurde keine Palladiumabscheidung beobachtet.

Die Ergebnisse sind in Tabelle 3 zusammengefaßt.

Tabelle 3

| Bsp. | Umsatz an 3-PSE [%] | TOF [$hr^{-1}$] | Carbonylierungs-Selektivität [%] | n-Selektivität [%] |
|---|---|---|---|---|
| 18 | 71 | 1770 | 92 | 80,6 |
| 19 | 95 | 2550 | >99 | 97,5 |
| 20 | 96 | 3990 | >99 | 98,4 |
| 3-PSE: 3-Pentensäuremethylester<br>TOF: Turn-over-frequency (mol Produkt / mol Katalysator/ Stunde) | | | | |

**Patentansprüche**

1. Zur Herstellung eines Katalysatorsystems für die Carbonylierung un olefinisch ungesättigten Verbindungen (X) mittels CO und einer seine Hydroxylgruppe enthaltenden Verbindung (XI) geeignete Verbindung der Formel (I)

$$L^1 - X - L_2 \qquad (I)$$

mit

X: niedere Alkylengruppe mit 1 - 6 Kohlenstoffatomen zwischen den beiden Resten $L_1$ und $L_2$, Arylengruppe oder Alkarylengruppe

$L^1$:

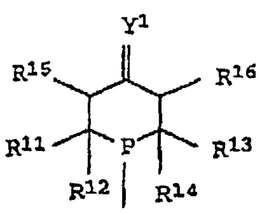

mit

| | |
|---|---|
| $Y^1$ | Sauerstoff, Schwefel oder N-$R^{17}$ |
| $R^{12}$, $R^{14}$, $R^{15}$, $R^{16}$, $R^{17}$ | unabhängig voneinander Wasserstoff, Alkyl oder Aryl |
| $R^{11}$, $R^{13}$ | unabhängig voneinander Alkyl oder Aryl |

$L^2$:

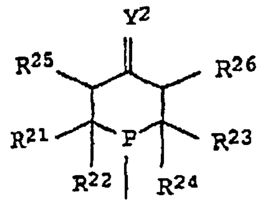

mit

| | |
|---|---|
| $Y^2$ | Sauerstoff, Schwefel oder N-$R^{27}$ |
| $R^{22}$, $R^{24}$, $R^{25}$, $R^{26}$, $R^{27}$ | unabhängig voneinander Wasserstoff, Alkyl oder Aryl |
| $R^{21}$, $R^{23}$ | unabhängig voneinander Alkyl oder Aryl |

wobei $L^1$ und $L^2$ gleich oder unterschiedlich sein können, und
wobei die Gruppen X, $R^{11}$-$R^{17}$ und $R^{21}$-$R^{27}$ Substituiert sein können.

**2.** Verbindung der Formel (I) nach Anspruch 1, wobei $L^1$ und $L^2$ gleich sind.

**3.** Verbindung der Formel (I) nach Anspruch 1 oder 2, wobei X eine niedere Alkylengruppe ist.

**4.** Verbindung der Formel (I) nach den Ansprüchen 1 bis 3, wobei X ausgewählt ist aus der Gruppe bestehend aus Methylen, 1,2-Ethylen, 1,3-Propylen, 1,4-Butylen.

**5.** Verbindung der Formel (I) nach den Ansprüchen 1 bis 4, wobei $Y^1$ und $Y^2$ Sauerstoff sind.

**6.** Verbindung der Formel (I) nach den Ansprüchen 1 bis 5, wobei $R^{11}$, $R^{12}$, $R^{13}$, $R^{14}$, $R^{15}$, $R^{16}$, $R^{17}$ ,$R^{21}$, $R^{22}$, $R^{23}$, $R^{24}$, $R^{25}$, $R^{26}$ und $R^{27}$ innerhalb der Definitionen gemäß Anspruch 1 unabhängig voneinander ausgewählt sind aus der Gruppe bestehend aus Wasserstoff, Methyl, Ethyl, n-Propyl, i-Propyl, n-Butyl, i-Butyl, s-Butyl, t-Butyl.

**7.** Verfahren zur Herstellung einer Verbindung der Formel (I) gemäß den Ansprüchen 1 bis 6, **dadurch gekennzeichnet, daß** man eine Verbindung der Formel (II)

$$H_2P - X - PH_2 \quad (II)$$

mit einer Verbindung der Formel (IV)

$$(R^{11} R^{12} C) = (C R^{15}) -(C=Y^1) - (CR^{16}) = (CR^{13} R^{14}) \quad (IV)$$

und einer Verbindung der Formel (V)

$$(R^{21} R^{22} C) = (C R^{25}) -(C=Y^2) - (C R^{26}) = (C R^{23} R^{24}) \quad (V)$$

umsetzt, wobei $R^{11}$, $R^{12}$, $R^{13}$, $R^{14}$,$R^{15}$, $R^{16}$, $R^{21}$, $R^{22}$, $R^{23}$, $R^{24}$, $R^{25}$, $R^{26}$, $Y^1$, $Y^2$ und X die in den Ansprüchen 1 bis 6 genannten Bedeutungen haben.

**8.** Verwendung einer Verbindung der Formel (I) gemäß den Ansprüchen 1 bis 6 als Bestandteil eines als Katalysator geeigneten Systems für die Carbonylierung von olefinisch ungesättigten Verbindungen (X) mittels CO und einer eine Hydroxylgruppe enthaltenden Verbindung (XI).

**9.** Als Katalysator für die Carbonylierung von olefinisch ungesättigten Verbindungen (X) mittels CO und einer eine

Hydroxylgruppe enthaltenden Verbindung (XI). geeignetes System (VI), erhältlich durch Umsetzung von

a) einer Quelle für ein Metall-Ion eines Metalls (VII) der VIII. Nebengruppe des Periodensystems der Elemente mit
b) einer Verbindung der Formel (I) gemäß den Ansprüchen 1 bis 6.

**10.** System (VI) nach Anspruch 9, wobei Metall (VII) ausgewählt ist aus der Gruppe bestehend aus Palladium, Platin, Rhodium und Iridium.

**11.** System (VI) nach Anspruch 9 oder 10, wobei man als Metall (VII) Palladium einsetzt.

**12.** System (VI) nach den Ansprüchen 9 bis 11, erhältlich in Gegenwart einer Anionenquelle (IX).

**13.** System (VI) nach Anspruch 12, wobei man als Anionenquelle (IX) eine Verbindung einsetzt, die in der Lage ist, unter Abspaltung eines $H^+$-Ions ein Anion zur Verfügung zu stellen.

**14.** Verfahren zur Carbonylierung von olefinisch ungesättigten Verbindungen (X) durch Umsetzung einer Verbindung der Formel (X) mit Kohlenmonoxid und einer eine Hydroxylgruppe enthaltenden Verbindung (XI) in Gegenwart eines Katalysators, **dadurch gekennzeichnet, daß** der Katalysator eine Verbindung der Formel (I) gemäß den Ansprüchen 1 bis 6 enthält, sowie eine Quelle für ein Metall-Ion eines Metalls (VII) enthält.

**15.** Verfahren zur Carbonylierung von olefinisch ungesättigten Verbindungen (X) durch Umsetzung einer Verbindung der Formel (X) mit Kohlenmonoxid und einer eine Hydroxylgruppe enthaltenden Verbindung (XI) in Gegenwart eines Katalysatorsystems, **dadurch gekennzeichnet, daß** der Katalysator ein System der Formel (VI) gemäß den Ansprüchen 9 bis 13 enthält.

**16.** Verfahren nach Anspruch 14 oder 15, wobei man als Verbindung der Formel (X) n-Pentensäure oder deren Derivate der Formel (XII)

$$C_4H_7 - R^{31} \qquad \text{(XII)}$$

wobei $R^{31}$: -CN oder $COOR^{32}$ mit $R^{32}$ Wasserstoff, Alkyl oder Aryl einsetzt.

**17.** Verfahren nach Anspruch 16, wobei $R^{32}$ ausgewählt ist aus der Gruppe bestehend aus Wasserstoff, Methyl, Ethyl, n-Propyl, i-Propyl, n-Butyl. i-Butyl, s-Butyl, t-Butyl.

**18.** Verfahren nach den Ansprüchen 14 bis 17, wobei man als Verbindung der Formel (XII) zu mindestens 80 Gew.-% 3-Pentennitril einsetzt.

**19.** Verfahren nach den Ansprüchen 14 bis 17, wobei man als Verbindung der Formel (XII) zu mindestens 80 Gew.-% 3-Pentensäuremethylester einsetzt.

## Claims

**1.** A compound of formula (I) suitable for the preparation of a catalyst system for the carbonylation of olefinically unsaturated compounds (X) using CO and a compound (XI) containing a hydroxyl group:

$$L^1 - X - L^2 \qquad \text{(I)}$$

in which

X is a lower alkylene group having 1-6 carbon atoms between the two radicals $L_1$ and $L_2$, an arylene group or an alkarylene group;

$L^1$ is

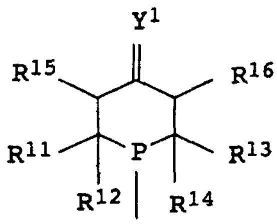

in which

| | |
|---|---|
| $Y^1$ | is oxygen, sulfur or N-$R^{17}$, |
| $R^{12}$, $R^{14}$, $R^{15}$, $R^{16}$ and $R^{17}$ | independently of one another being hydrogen, alkyl or aryl; and |
| $R^{11}$ and $R^{13}$ | independently of one another being alkyl or ayrl, |
| $L^2$ | is |

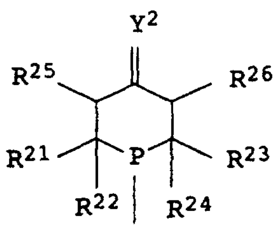

in which

| | |
|---|---|
| $Y^2$ | is oxygen, sulfur or N-$R^{27}$, |
| $R^{22}$, $R^{24}$, $R^{25}$, $R^{26}$ and $R^{27}$ | independently of one another being hydrogen, alkyl or aryl, |
| $R^{21}$ and $R^{23}$ | independently of one another being alkyl or aryl, |

it being possible for $L^1$ and $L^2$ to be identical or different, and it being possible for the groups, X, $R^{11}$-$R^{17}$ and $R^{21}$-$R^{27}$ to be substituted.

**2.** A compound of formula (I) as claimed in claim 1 in which $L^1$ and $L^2$ are identical.

**3.** A compound of formula (I) as claimed in claim 1 or 2 in which X is a lower alkylene group.

**4.** A compound of formula (I) as claimed in any of claims 1 to 3 in which X is selected from the group comprising methylene, 1,2-ethylene, 1,3-propylene and 1,4-butylene.

**5.** A compound of formula (I) as claimed in any of claims 1 to 4 in which $Y^1$ and $Y^2$ are oxygen.

**6.** A compound of formula (I) as claimed in any of claims 1 to 5 in which $R^{11}$, $R^{12}$, $R^{13}$, $R^{14}$, $R^{15}$, $R^{16}$, $R^{17}$, $R^{21}$, $R^{22}$, $R^{23}$, $R^{24}$, $R^{25}$, $R^{26}$ and $R^{27}$, within the definitions as claimed in claim 1, independently of one another are selected from the group comprising hydrogen, methyl, ethyl, n-propyl, i-propyl, n-butyl, i-butyl, s-butyl and t-butyl.

**7.** A process for the preparation of a compound of formula (I) as claimed in any of claims 1 to 6, wherein a compound of formula (II):

$$H_2P - X - PH_2 \quad (II)$$

is reacted with a compound of formula (IV):

$$(R^{11} R^{12} C) = (C R^{15}) -(C=Y^1) - (CR^{16}) = (CR^{13} R^{14}) \quad (IV)$$

and a compound of formula (V):

$$(R^{21} R^{22} C) = (C R^{25}) -(C=Y^2) - (C R^{26}) = (C R^{23} R^{24}) \quad (V)$$

$R^{11}$, $R^{12}$, $R^{13}$, $R^{14}$, $R^{15}$, $R^{16}$, $R^{21}$, $R^{22}$, $R^{23}$, $R^{24}$, $R^{25}$, $R^{26}$, $Y^1$, $Y^2$ and X being as defined in any of claims 1 to 6.

**8.** The use of a compound of formula (I) as claimed in any of claims 1 to 6 as a constituent of a system suitable as a catalyst for the carbonylation of olefinically unsaturated compounds (X) using CO and a compound (XI) containing a hydroxyl group.

**9.** A system (VI) suitable as a catalyst for the carbonylation of olefinically unsaturated compounds (X) using CO and a compound (XI) containing a hydroxyl group, obtainable by reacting

a) a source of an ion of a metal (VII) of subgroup VIII of the Periodic Table of the Elements with

b) a compound of formula (I) as claimed in any of claims 1 to 6.

**10.** A system (VI) as claimed in claim 9 in which the metal (VII) is selected from the group comprising palladium, platinum, rhodium and iridium.

**11.** A system (VI) as claimed in claim 9 or 10 in which the metal (VII) used is palladium.

**12.** A system (VI) as claimed in any of claims 9 to 11 obtainable in the presence of an anion source (IX).

**13.** A system (VI) as claimed in claim 12 in which the anion source (IX) used is a compound capable of generating an anion by elimination of an $H^+$ ion.

**14.** A process for the carbonylation of olefinically unsaturated compounds (X) by reacting a compound of formula (X) with carbon monoxide and a compound (XI) containing a hydroxyl group, in the presence of a catalyst, wherein the catalyst contains a compound of formula (I) as claimed in any of claims 1 to 6, and also contains a source of an ion of a metal (VII).

**15.** A process for the carbonylation of olefinically unsaturated compounds (X) by reacting a compound of formula (X) with carbon monoxide and a compound (XI) containing a hydroxyl group, in the presence of a catalyst system, wherein the catalyst contains a system of formula (VI) as claimed in any of claims 9 to 13.

**16.** A process as claimed in claim 14 or 15 in which the compound of formula (X) used is n-pentenoic acid or derivatives thereof of formula (XII):

$$C_4H_7 - R^{31} \quad (XII)$$

in which $R^{31}$ is -CN or $COOR^{32}$, where $R^{32}$ is hydrogen, alkyl or aryl.

**17.** A process as claimed in claim 16 in which $R^{32}$ is selected from the group comprising hydrogen, methyl, ethyl, n-propyl, i-propyl, n-butyl, i-butyl, s-butyl and t-butyl.

**18.** A process as claimed in any of claims 14 to 17 in which at least 80% by weight of the compound of formula (XII)

used is 3-pentene nitrile.

**19.** A process as claimed in any of claims 14 to 17 in which at least 80% by weight of the compound of formula (XII) used is methyl 3-pentenoate.

## Revendications

**1.** Composé approprié à la préparation d'un système catalytique pour la carbonylation de composés oléfiniquement insaturés (X) au moyen de CO et d'un composé (XI) contenant des groupes hydroxyle, et présentant la formule (I)

$$L^1\text{-}X\text{-}L^2 \qquad (I)$$

dans laquelle

X représente des groupes alkylène inférieurs comportant de 1 6 atomes de carbone entre les deux restes L1 et $L_2$, des groupes arylène ou des groupes alkarylène,

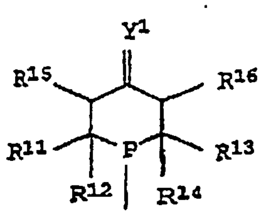

$L^1$ représente

avec

| | |
|---|---|
| $Y^1$ | représentant de l'oxygène, du soufre ou N-$R^{17}$, |
| $R^{12}$, $R^{14}$, $R^{15}$; $R^{16}$, $R^{17}$ | représentant indépendamment les uns des autres de l'hydrogène ou un radical alkyle ou aryle |
| $R^{11}$, $R^{13}$ | représentant indépendamment les uns des autres un radical alkyle ou aryle, |

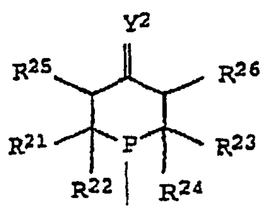

$L^2$ représente

avec

| | |
|---|---|
| $Y^2$ | représentant de l'oxygène, du soufre ou N-$R^{27}$ |
| $R^{22}$, $R^{24}$ $R^{25}$, $R^{26}$, $R^{27}$ | représentant indépendamment les uns des autres de l'hydrogène ou un radical alkyle ou aryle, |
| $R^{21}$, $R^{23}$ | représentant indépendamment l'un de l'autre un radical alkyle ou aryle, |

où $L^1$ et $L^2$ peuvent être identiques ou différents et où les groupes X, $R^{11}$ à $R^{17}$ et $R^{21}$ à $R^{27}$ peuvent être substitués.

**2.** Composé de formule (I) selon la revendication 1, dans lequel $L^1$ et $L^2$ sont identiques.

**3.** Composé de formule (I) selon la revendication 1 ou 2, dans lequel X est un groupe alkylène inférieur.

**4.** Composé de formule (I) selon les revendications 1 à 3, dans lequel X est choisi dans le groupe formé par des radicaux méthylène, 1,2-éthylène, 1,3-propylène, 1,4-butylène.

**5.** Composé de formule (I) selon les revendications 1 à 4, dans lequel $Y^1$ et $Y^2$ représentent de l'oxygène.

**6.** Composé de formule (I) selon les revendications 1 à 5, dans lequel $R^{11}$, $R^{12}$, $R^{13}$, $R^{14}$, $R^{15}$, $R^{16}$, $R^{17}$, $R^{21}$, $R^{22}$, $R^{23}$, $R^{24}$, $R^{25}$, $R^{26}$ et $R^{27}$, dans le cadre des définitions selon la revendication 1, sont choisis indépendamment les uns des autres dans le groupe formé par l'hydrogène et des radicaux méthyle, éthyle, n-propyle, i-propyle, n-butyle, i-butyle, s-butyle, t-butyle.

**7.** Procédé de préparation d'un composé de formule (I) selon les revendications 1 à 6, **caractérisé en ce que** l'on fait réagir un composé de formule (II)

$$H_2P - X - PH_2 \qquad (II)$$

avec un composé de formule (IV)

$$(R^{11}\ R^{12}\ C) = (C\ R^{15}) - (C{=}Y^1) - (CR^{16}) = (CR^{13}R^{14}) \qquad (IV)$$

et un composé de formule (V)

$$(R^{21}\ R^{22}\ C) = (C\ R^{25}) - (C{=}Y^2) - (C\ R^{26}) = (C\ R^{23}\ R^{24}) \qquad (V)$$

où $R^{11}$, $R^{12}$, $R^{13}$, $R^{14}$, $R^{15}$, $R^{16}$, $R^{21}$, $R^{22}$, $R^{23}$, $R^{24}$, $R^{25}$, $R^{26}$, $Y^1$, $Y^2$ et X ont les significations indiquées dans les revendications 1 à 6.

**8.** Utilisation d'un composé de formule (I) selon les revendications 1 à 6 comme constituant d'un système convenant comme catalyseur pour la carbonylation de composés oléfiniquement insaturés (X) au moyen de CO et d'un composé (XI) contenant des groupes hydroxyle.

**9.** Système (VI) convenant comme catalyseur pour la carbonylation de composés oléfiniquement insaturés (X) au moyen de CO et d'un composé (XI) contenant des groupes hydroxyle, que l'on peut obtenir par réaction de

a) une source d'un ion métallique d'un métal (VII) du VIIIème sous-groupe du système périodique des éléments avec
b) un composé de formule (I) selon les revendications 1 à 6.

**10.** Système (VI) selon la revendication 9, dans lequel le métal (VII) est choisi dans le groupe formé par le palladium, le platine, le rhodium et l'iridium.

**11.** Système (VI) selon la revendication 9 ou 10, dans lequel on met en oeuvre du palladium en tant que métal (VII).

**12.** Système (VI) selon les revendications 9 à 11, que l'on peut obtenir en présence d'une source d'anions (IX).

**13.** Système (VI) selon la revendication 12, dans lequel on met en oeuvre comme source d'anions un composé capable de procurer un anion avec libération d'un ion $H^+$.

**14.** Procédé de carbonylation de composés oléfiniquement insaturés (X) par réaction d'un composé de formule (X) avec du monoxyde de carbone et un composé (XI) contenant un groupe hydroxyle en présence d'un catalyseur, **caractérisé en ce que** le catalyseur contient un composé de formule (I) selon les revendications 1 à 6, ainsi qu'une

source d'un ion métallique d'un métal (VII).

**15.** Procédé de carbonylation de composés oléfiniquement insaturés (X) par réaction d'un composé de formule (X) avec du monoxyde de carbone et d'un composé (XI) contenant un groupe hydroxyle en présence d'un système catalytique, **caractérisé en ce que** le catalyseur contient un système de formule (VI) selon les revendications 9 à 13.

**16.** Procédé selon la revendication 14 ou 15, dans lequel on met en oeuvre comme composé de formule (X) de l'acide n-penténique ou ses dérivés de formule (XII)

$$C_4H_7\text{-}R^{31} \qquad \text{(XII)}$$

dans laquelle $R^{31}$ représente -CN ou $COOR^{32}$, avec $R^{32}$ représentant de l'hydrogène ou un radical alkyle ou aryle.

**17.** Procédé selon la revendication 16, dans lequel $R^{32}$ est choisi dans le groupe formé par de l'hydrogène et des radicaux méthyle, n-propyle, i-propyle, n-butyle, i-butyle, s-butyle, t-butyle.

**18.** Procédé selon les revendications 14 à 17, dans lequel on met en oeuvre comme composé de formule (XII) du nitrile 3-penténique à raison d'au moins 80% en poids.

**19.** Procédé selon les revendications 14 à 17, dans lequel on met en oeuvre comme composé de formule (XII) du méthylester d'acide 3-penténique à raison d'au moins 80% en poids.